# EUROPEAN PATENT APPLICATION

(11) **EP 2 796 151 A1**
(43) Date of publication of application: **29.10.2014**
(21) Application number: 13776300.9
(22) Date of filing: 01.04.2013
(51) Int. Cl.: A61K 47/48, A61K 9/16, A61K 9/14

(54) **CERIA NANOCOMPLEX, PHARMACEUTICAL COMPOSITION COMPRISING SAID CERIA NANOCOMPLEX, AND METHOD FOR PREPARING SAME**

(30) Priority: 09.04.2012 KR 20120036880
(71) Applicant: SNU R&DB Foundation, Seoul 151-742 (KR)
(72) Inventor: HYEON, Taeghwan, Seoul 135-537 (KR); LEE, Seung-Hoon, Seoul 110-744 (KR); KIM, Taeho, Seoul 157-794 (KR); KIM, Chi Kyung, Seoul 143-770 (KR)
(74) Representative: Bassil, Nicholas Charles
(86) International application number: PCT/KR2013/002665
(87) International publication number: WO 2013/154290

(57) **Abstract**

The present invention relates to ceria nanocomposite, pharmaceutical composition comprising the ceria nanocomposite, and method for preparing same. More particularly, the present invention is directed to a ceria nanocomposite comprising a ceria nanoparticle, wherein the ceria nanoparticle is encapsulated with a surfactant and the surfactant is encapsulated with polyethylene glycol-phospholipid, pharmaceutical composition for preventing or treating ischemic stroke comprising thereof and method for preparing the same.

## Description

### Technical Field

The present invention relates to ceria nanocomposite, pharmaceutical composition comprising, and method for preparing the same. More particularly, the present invention is directed to a ceria nanocomposite comprising a ceria nanoparticle, wherein the ceria nanoparticle is encapsulated with a surfactant and the surfactant is encapsulated with polyethylene glycol-phospholipid, a pharmaceutical composition for preventing or treating ischemic stroke comprising thereof, and method for preparing the same.

### Background Art

Ischemic stroke is the leading cause of adult disability in US and the second leading cause of death worldwide. Reactive oxygen species (ROS) such as superoxide anion (O₂^{•-}), hydrogen peroxide (H₂O₂), and hydroxyl radical (HO^{•-}) are generated and accumulate during the ischemic periods, and induce oxidative damages, which is one of the most critical mechanisms responsible for ischemic stroke damage. However, there has been no proven neuroprotective therapy for ischemic stroke in clinical practice.

Ceria nanoparticles have been known to exhibit free radical scavenging activity by reversibly binding oxygen and shifting between Ce³⁺ (reduced) and Ce⁴⁺ (oxidized) at the particle surface (Celardo, I., Pedersen, J. Z., Traversa, E. & Ghibelli, L. Pharmacological potential of cerium oxide nanoparticles. Nanoscale 3, 1411-1420, 2011).

Recently, it was reported that ceria nanoparticles exhibit superoxide dismutase-mimetic activity (when cerium is oxidized to Ce⁴⁺) and catalase-mimetic activity (when cerium is reduced to Ce³⁺) to protect cells against two dominant ROS, superoxide anion (O₂^{•-}) and hydrogen peroxide (H₂O₂) (Heckert, E. G., Karakoti, A. S., Seal, S. & Self, W. T. The role of cerium redox state in the SOD mimetic activity of nanoceria. Biomaterials 29, 2705-2709, 2008; Korsvik, C., Patil, S., Seal, S. & Self, W. T. Superoxide dismutase mimetic properties exhibited by vacancy engineered ceria nanoparticles. Chem. Commun. 1056-1058, 2007).

PCT/US2006/024963 discloses cerium oxide nanoparticles for use in therapeutic composition, and describes that the therapeutic compositions may be used for treating stroke. However, PCT/US2006/024963 does not describe any example that cerium oxide nanoparticles were applied to treat stroke.

Estevez et al. discloses that ceria nanoparticle may be used for treatment of stroke (A Y Estevez, S Pritchard, K Harper, J W Aston, A Lynch, J J Lucky, J S Ludington, P Chatani, W P Mosenthal, J C Leiter, S Andreescu, J S Erlichman, "Neuroprotective mechanisms of cerium oxide nanoparticles in a mouse hippocampal brain slice model of ischemia", FREE RADICAL BIOLOGY AND MEDICINE, Volume: 51, Issue: 6, Pages: 1155-1163, 2011).

However, Estevez et al. does not disclose ceria nanocomposites, but only ceria nanoparticles.

The present inventors have paid attention to the fact that ceria nanocomposite prepared by encapsulating ceria nanoparticles with a surfactant such as oleylamine and capping them with PEG-phospholipid, easily penetrate cell membranes without toxicity and effectively diffuse into cytoplasm, and thus have completed the present invention.

In addition, the present inventors have revealed that the ceria nanocomposite of the present invention is effective to treat and prevent ischemic stroke, and accordingly have invented a pharmaceutical composition for treating or preventing ischemic stroke, which comprises the ceria nanocomposite.

### Disclosure

### Technical Problem

The primary object of the present invention is to provide a ceria nanocomposite comprising a ceria nanoparticle, wherein the ceria nanoparticle is encapsulated with a surfactant and the surfactant is encapsulated with polyethylene glycol-phospholipid.

Another object of the present invention is to provide a pharmaceutical composition for treating or preventing ischemic stroke, comprising a ceria nanocomposite which comprises a ceria nanoparticle, wherein the ceria nanoparticle is enclosed with a surfactant and the surfactant is capped with polyethylene glycol-phospholipid.

Yet another object of the present invention is to provide method for preparing of ceria nanocomposite (i) reacting a cerium precursor with a surfactant in a first organic solvent to form a cerium-surfactant complex; adding water to the cerium-surfactant complex solution of the step (i), while heating the solution, to form a cerium nanoparticle enclosed with the surfactant; and (iii) encapsulating the surfactant-capped ceria nanoparticle with polyethylene glycol-lipid in a second organic solvent.

### Technical Solution

The primary object of present invention can be accomplished by providing a ceria nanocomposite comprising a ceria nanoparticle, wherein the ceria nanoparticle is encapsulated with a surfactant and the surfactant is encapsulated with polyethylene glycol-phospholipid.

As used herein, the term "phospholipid" refers to an amphipathic molecule that includes a lipid region and hydrophilic region having phosphrus. Preferably the hydrophilic region may be phosphatidylserine, phosphatidylethanolamine, phosphatidylcholine or phosphatidylinositol.

As used herein, the term "polyethylene glycol (PEG)" refers to a polymeric compound that has a molecular weight of about 100 Da to about 10,000 Da according to the number of ethylene oxide in the polymer chain.

As used herein, the term "PEG-phospholipid" is a compound of the following chemical formula I.

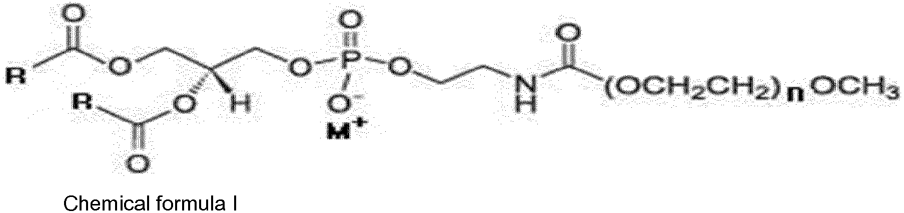

In the chemical formula I, R is C₁₅-C₂₅ linear alkyl, M⁺ is NH⁴⁺ or Na⁺, and n is an integer of 35 to 55.

The ceria nanocomposite of the present invention has a structure that a hydrophilic region of the surfactant chemically or physically binds to the surface of the ceria nanoparticle and two strands of alkyl (R) at the phospholipid region of the PEG-phospholipid encapsulate the lipophilic region of the surfactant.

In the ceria nanocomposite of present invention, a size of the ceria nanoparticle is preferably 3 nm - 20 nm, and a size of the ceria nanocomposite is preferably 15 nm - 100 nm.

In addition, in the ceria nanocomposite of the present invention, the surfactant may be C₈-C₂₅ alkyl trimethyl ammonium salt ((CH₃)₃RNX, R is C₈ ∼ C₂₅, X is Br, Cl, I) such as cetyltrimethylammonium bromide (CTAB), octyltrimethylammonium bromide (OCTAB) or dodecyltrimethylammonium bromide (DTAB); C₆-C₂₀ alkylamine such as oleylamine, octylamine, hexadecylamine or octadecylamine; (poly(ethylene oxide))-(poly(propylene oxide))-(poly(ethylene oxide)) (PEO-PPO-PEO)-based poly(alkylene oxide) triblock copolymer such as (ethylene oxide)₂₀(propylene oxide)₇₀(ethylene oxide)₂₀ ; or alkali salts of C₁₂-C₁₈ fatty acid including unsaturated fatty acids (oleic acid (C₁₈), linoleic acid (C₁₈)) and saturated fatty acids (lauric acid (C₁₂), myristic acid (C₁₄), palmitic acid (C₁₆), stearic acid (C₁₈).

Another object of the present invention is achieved by providing a pharmaceutical composition for treating or preventing ischemic stroke, comprising a ceria nanocomposite which comprises a ceria nanoparticle, wherein said ceria nanoparticle is enclosed with a surfactant and said surfactant is capped with polyethylene glycol-phospholipid.

In the pharmaceutical composition comprising the ceria nanocomposite of present invention, a size of the ceria nanoparticle is preferably 3 nm - 20 nm, and a size of the ceria nanocomposite is preferably 15 nm - 100 nm.

In the pharmaceutical composition comprising the ceria nanocomposite of present invention, the surfactant may be C₈-C₂₅ alkyl trimethyl ammonium salt ((CH₃)₃RNX, R is C₈ - C₂₅, X is Br, Cl, I) such as cetyltrimethylammonium bromide (CTAB), octyltrimethylammonium bromide (OCTAB) or dodecyltrimethylammonium bromide (DTAB); C₆-C₂₀ alkylamine such as oleylamine, octylamine, hexadecylamine or octadecylamine; (poly(ethylene oxide))-(poly(propylene oxide))-(poly(ethylene oxide)) (PEO-PPO-PEO)-based poly(alkylene oxide) triblock copolymer such as (ethylene oxide)₂₀(propylene oxide)₇₀(ethylene oxide)₂₀ ; or alkali salts of C₁₂-C₁₈ fatty acid including unsaturated fatty acids (oleic acid (C₁₈), linoleic acid (C₁₈)) and saturated fatty acids (lauric acid (C₁₂), myristic acid (C₁₄), palmitic acid (C₁₆), stearic acid (C₁₈).

In addition, the pharmaceutical composition comprising the ceria nanocomposite of present invention, the ceria nanocomposite is preferably contained by 0.5 mg - 0.7 mg per 1 kg of subject to be administered.

Further, administration routes of the pharmaceutical composition for preventing or treating ischemic stroke of the present invent may be oral, nasal, intravenous, rectum, intraperitoneal, hypodermic, or intradermal.

Yet another object of the present invention can be achieved by providing a method for preparing a ceria nanocomposite, comprising: (i) reacting a cerium precursor with a surfactant in a first organic solvent to form a cerium-surfactant complex; (ii) adding water to said cerium-surfactant complex solution of the step (i), while heating said solution, to form a cerium nanoparticle enclosed with said surfactant; and (iii) encapsulating said surfactant-capped ceria nanoparticle with polyethylene glycol-lipid in a second organic solvent.

The first organic solvent may be aromatic compounds such as xylene, toluene, mesitylene, etc.; ether compouds such as octyl ether, butyl ether, hexyl ether, decyl ether, etc.; heterocyclic compounds such as pyridine, etc.; dimethylsulfoxide (DMSO), dimethylformamide (DMF); alchols such as octanol, decanol, etc.; hydrocarbons such as decane, dodecane, tetradecane, hexadecane, etc.

The cerium precursor may be selected from cerium (III) acetate hydrate, cerium (III) acetylacetonate hydrate, cerium (III) carbonate hydrate, cerium (IV) hydroxide, cerium (III) fluoride, cerium (IV) fluoride, cerium (III) chloride, cerium (III) chloride heptahydrate, cerium (III) bromide, cerium (III) iodide, cerium (III) nitrate hydrate, cerium (III) oxalate hydrate, cerium (III) sulfate, cerium (III) sulfate hydrate or cerium (IV) sulfate.

The surfactant may be C₈-C₂₅ alkyl trimethyl ammonium salt ((CH₃)₃RNX, R is C₈ ∼ C₂₅, X is Br, Cl, I) such as cetyltrimethylammonium bromide (CTAB), octyltrimethylammonium bromide (OCTAB) or dodecyltrimethylammonium bromide (DTAB); C₆-C₂₀ alkylamine such as oleylamine, octylamine, hexadecylamine or octadecylamine; (poly(ethylene oxide))-(poly(propylene oxide))-(poly(ethylene oxide)) (PEO-PPO-PEO)-based poly(alkylene oxide) triblock copolymer such as (ethylene oxide)₂₀(propylene oxide)₇₀(ethylene oxide)₂₀ ; or alkali salts of C₁₂-C₁₈ fatty acid including unsaturated fatty acids (oleic acid (C₁₈), linoleic acid (C₁₈)) and saturated fatty acids (lauric acid (C₁₂), myristic acid (C₁₄), palmitic acid (C₁₆), stearic acid (C₁₈).

It is preferable that a heating temperature of the step (ii) is 80°C to 90°C, and a heating time of the step (ii) is 30 min to 3hr.

The second organic solvent of the step (iii) may be chloroform, dichloromethane, pentane, hexane, heptane, cyclohexane, ethyl acetate, tetrahydrofuran, diethyl ether or trichloroethylene.

The ceria nanocomposite prepared in the step (iii) is preferably a size of 15 nm to 100 nm.

### Advantageous Effects

The ceria nanocomposite of the present invention may efficiently reach efficiently into the cytoplasm through cellular membranes. Therefore, the ceria nanocomposite of the present invention exhibits efficient treatment effects on the region where ischemic stroke has occurred.

### Brief Description of the Drawings

Fig. 1 shows the characteristics of the ceria nanoparticles prepared in Example 1 of the present invention.
Fig. 2 shows the hydrodynamic size distribution of the ceria nanoparticles encapsulated with PEG-phospholipid of Example 2 (Fig. 2a), and an image of aqueous solution in which the nanoparticles are dispersed (Fig. 2b).
Fig. 3 shows the color change of the ceria nanoparticles-dispersed solution, the color change after addition of hydrogen peroxide solution to the solution, and the UV/Vis absorption of the ceria nanoparticles prepared in Example 3 of the present invention.
Fig. 4 shows the infarct area and the ischemic cell death in the focal ischemia-reperfusion model of Example 4 of the present invention.
Fig. 5 shows the change of cell viability according to the concentration of the added ceria nanoparticle under the oxidative damage of cells in Example 5 of the present invention.
Fig. 6 shows fluorescence microscopy images of the CHO-K1 cells incubated with ceria nanoparticles conjugated with fluorescent rhodamine dye, in Example 5 of the present invention.
Fig. 7 shows the biodistribution of the ceria nanoparticles in Example 6 of the present invention.
Fig. 8 shows ROS-scavenging and apoptosis-decreasing effects in Example 7 of the present invention.

### Best Mode for Carrying Out the Invention

Hereinafter, the present invention will be described in greater detail with reference to the following examples and drawings. The examples and drawings are given only for illustration of the present invention and not to be limiting the present invention.

### Example 1. Synthesis of ceria nanoparticles (reverse-micelle method)

1 mmol (0.43g) of cerium(III) acetate and 12 mmol (3.25g) of oleylamine (C₁₈ contents: about 80%-90%, Acros Organics) were added to 15 ml of xylene (98.5%, Aldrich). The resulting solution was stirred for 2 hr at room temperature and heated to 90°C under vacuum. 1 mL of deionized (DI) water was injected into the solution under a vigorous stirring at 90°C, and the observed color change to off-white demonstrated that the reaction has been occurred. The resulting mixture was aged at 90°C for 3 hr to give a light yellow colloidal solution, and cooled down to room temperature. Ethanol (100 ml) was added to the mixture to precipitate the ceria nanoparticles. The precipitate was washed with ethanol and acetone by centrifugation, and the resulting ceria nanoparticles were well dispersed in organic solvents such as n-hexane and chloroform.

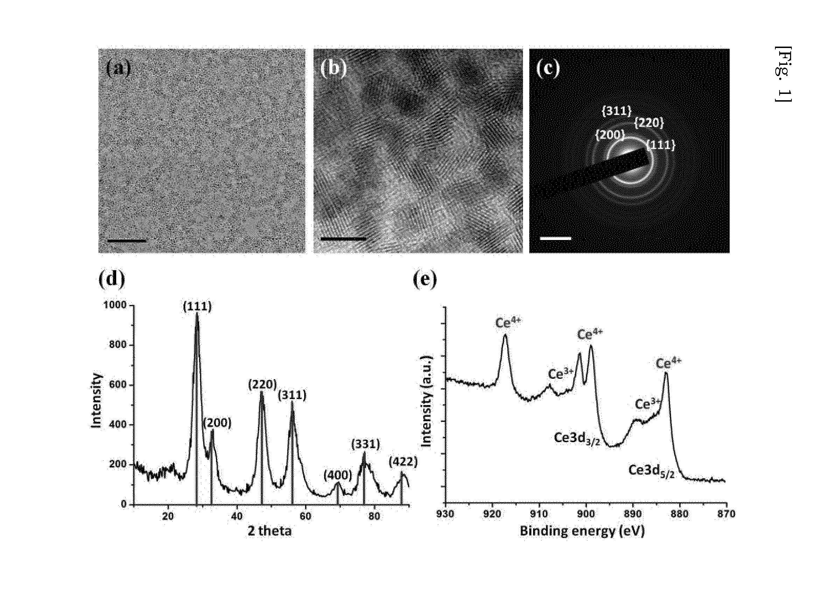

Transmission electron microscope (TEM) image revealed discrete and uniform 3 nm-sized ceria nanoparticles (Fig. 1a). High-resolution TEM image showed that they exhibited a cross-lattice pattern, demonstrating the highly crystalline nature despite of the low reaction temperature (Fig. 1b). The selected area electron diffraction (SAED) pattern (Fig. 1c) and X-ray diffraction (XRD) pattern (Fig. 1d) revealed the cubic fluorite structure (JCPDS card no. 34-0394). The particle size estimated by the Scherrer formula was 3.3 nm, which matched very well with that measured by TEM. In contrast to the white color of pure ceria nanoparticles, as-prepared nanoparticles exhibited yellowish color because they are composed of not only cerium (IV) oxide but also cerium (III) oxide. X-ray photoelectron spectroscopy (XPS) analysis were used to identify valence state of Ce³⁺ (peaks at 885.0 and 903.5 eV) and Ce⁴⁺ (peaks at 882.1, 888.1, 898.0, 900.9, 906.4, and 916.40 eV), and confirmed the mixed valence state as shown in Fig. 1e.

### Example 2. Synthesis of phospholipid-PEG capped ceria nanoparticles

In order to prepare biocompatible ceria nanoparticle, PEG processes were performed. 5 ml of ceria nanoparticles in CHCl₃ (10 mg/ml) was mixed with 5 ml of CHCl₃ solution containing 50 mg of 1,2-distearoyl-sn-glycero-3-phosphoethanolamine-N-[methoxy(polyethylene glycol)-2000] in the ratio of 1:1. Solvents were evaporated by rotary evaporator and, then incubated at 80°C in vacuum for 1 hr. The addition of 5 ml water resulted in a clear and light-yellowish suspension. After filtration, excess mPEG-2000 PE was removed by ultracentrifugation. The purified phospholipid-PEG capped ceria nanoparticles were dispersed in distilled water.

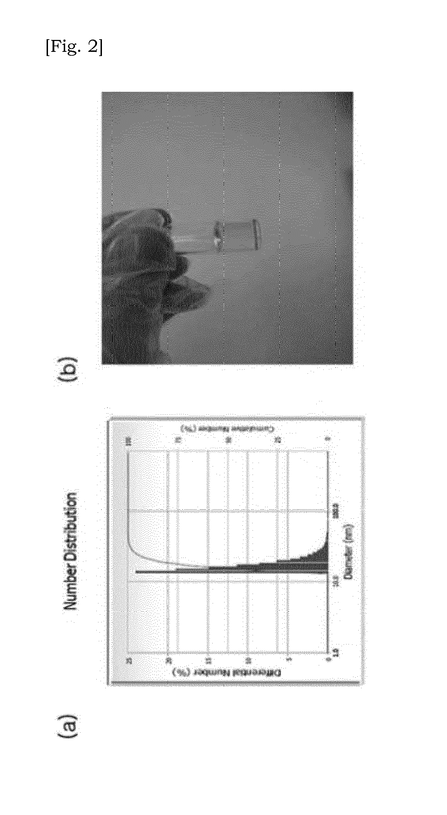

The phospholipid-PEG capped nanoparticles showed excellent colloidal stability in phosphate-buffered saline (PBS), and their hydrodynamic diameters were maintained at 18 nm for several weeks (Fig. 2).

### Example 3. Autocatalytic activity assay

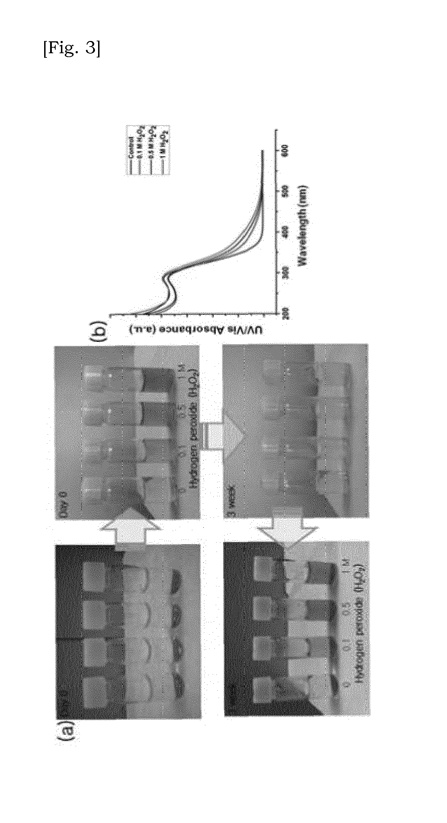

The autocatalytic activity assay was assessed by treating various concentration of hydrogen peroxide solution (H₂O₂) (0 M, 0.1 M, 0.5 M, and 1 M) into 5 mM ceria nanoparticle suspensions. Color changes from the suspensions were monitore, and UV/Vis absorbances were measured after the addition of hydrogen peroxide solution (Fig.3). As a control, a nanoparticles suspension without hydrogen peroxide was used. When hydrogen peroxide solutions of various concentrations were added to PBS solutions with 5 mM ceria nanoparticles, reversible autocatalytic activity persisted even after 3 weeks.

### Example 4. Focal ischemia-reperfusion model

Focal cerebral ischemia-perfusion was induced by using a minor modification of the endovascular internal carotid artery (ICA) suture method developed by Longa et al. After inhalation anesthesia using 3% isoflurane in 30% oxygen and 70% air, the left common carotid artery (CCA) was exposed at its bifurcation by a midline cervical incision. The external carotid artery (ECA), the ICA, and the CCA were ligated with a 5-0 silk suture. The CCA was then transected, and a 5-0 nylon monofilament suture (with its tip rounded by heating) was inserted into the CCA. To occlude the origins of the MCA and proximal anterior cerebral artery, the suture was advanced into the ICA for a distance of 20 mm. The suture was secured in place with a ligature, and the wound was closed. The monofilament was removed 60 minutes after occlusion. Rectal temperature was maintained at 37±0.5°C by using a thermistor-controlled heating blanket.

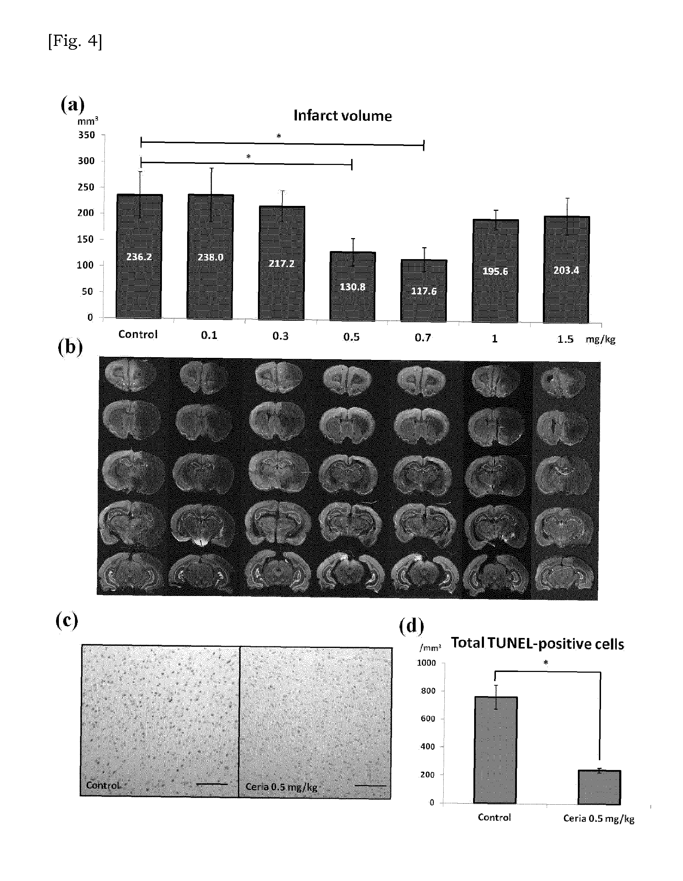

Immediately after reperfusion, various doses of ceria nanoparticle (0.1, 0.3, 0.5, 0.7, 1.0, 1.5 mg/kg) or phosphate-buffered saline (PBS) were intravenously injected and, then, optimal doses were detected by comparing brain infarct volumes in rats (Fig. 4a, 4b). Low-dose ceria nanoparticles (0.1 and 0.3 mg/kg) did not decrease infarct volumes, whereas ceria nanoparticles with concentrations of 0.5 and 0.7 mg/kg considerably reduced infarct volumes up to 50% of those of the control group (*p*<0.05). However, higher dose of ceria nanoparticles (1.0 and 1.5 mg/kg) failed to show protective effect from stroke. To support these findings, microscopic analysis of cell death in the brain slices with frozen section was performed by using terminal deoxynucleotidyl transferase-mediated dUTP-biotin nick end labeling (TUNEL) (Fig. 4c). In the quantitative analysis, the number of TUNEL-positive cells were remarkably decreased in the ceria-injected group (0.5 mg/kg) (p<0.05; Fig. 4d). Thus, it can be understood that optimal dose of ceria nanoparticles (0.5 to 0.7 mg/kg) has powerful neuroprotective effects in rodent stroke model.

### Example 5. Induction of cellular ROS production with tBHP and cell viability assay.

CHO-K1 cells in the 96-well plates were washed with phosphate buffered saline (PBS) 2 times, and 1mM tert-butyl hydroperoxide (tBHP) solutions were added and, then, were incubated for 1 hour. After washing with phosphate buffered saline (PBS), ceria nanoparticles (0.125, 0.25 mM) were added into each well. 6 hours after treatment of the ceria nanoparticles, MTT (3-[4,5-dimethylthialzol-2-yl]-2,5-diphenyltetrazolium bromide) assay (Sigma) was performed.

To examine the cell viability, MTT (3-[4,5-dimethylthialzol-2-yl]-2,5-diphenyltetrazolium bromide) assay (Sigma) was used. CHO-K1 cells were initially seeded in triplicate to 96-well plates at a density of 25,000 cells per well and were grown in culture medium for 24 hr at 37 °C. CHO-K1 cells were washed with phosphate buffered saline (PBS) 2 times, and the various concentrations of ceria nanoparticles (0.125, 0.25, 0.5 mM) were added into each well. 6 hours after treatment of the ceria nanoparticles, the cells in the 96-well plates were assayed for viability. 20 µl of a 5mg/ml solution of MTT was added to each well, and they were incubated for 2hr at 37°C in a humidified 5% CO₂ atmosphere. The supernatant was aspirated and the cells dissolved in 200 µl of DMSO. After shaking for 30 minutes, the absorbance at 540nm was measured using a 96-well plate reader.

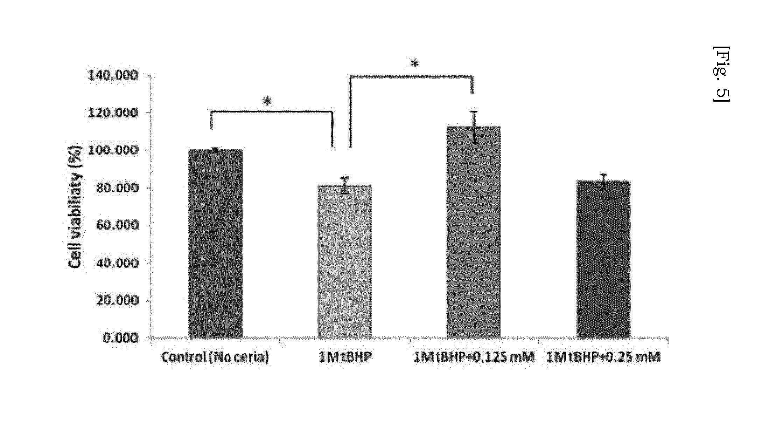

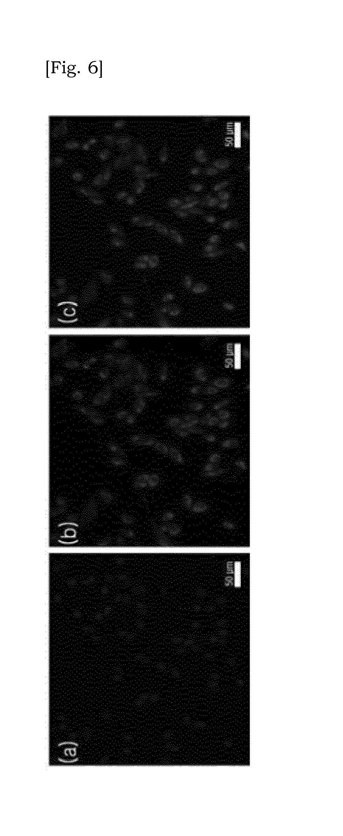

In the MTT assay, 1 M tBHP significantly increased cell death as compared with control (83%, p<0.05; Fig. 5), but addition of 0.125 mM of ceria nanoparticles reversed the cell viability (113%, p<0.05). It was also found that ceria nanoparticles were mostly detected in the intracellular spaces shown by fluorescence imaging with Rhodamine dye (Fig. 6). It can be understood that nanoparticles smaller than 50 nm, coated with lipophilic polymers, are efficiently diffused trough cellular membranes and located throughout the cytoplasm. These results demonstrate that the ceria nanoparticles introduced into the cells have protective effects for ROS-induced cell death *in vitro.*

### Example 6. Biodistribution of ceria nanoparticles.

6 to 24 hours after infusion of the ceria nanoparticle, the rats with focal cerebral ischemia were decapitated to rapidly harvest the brain and other organs. Post-mortem samples were obtained to determine the ceria concentration in brain, heart, kidney, liver, and blood by inductively coupled plasma-mass spectrometry (ICP-MS).

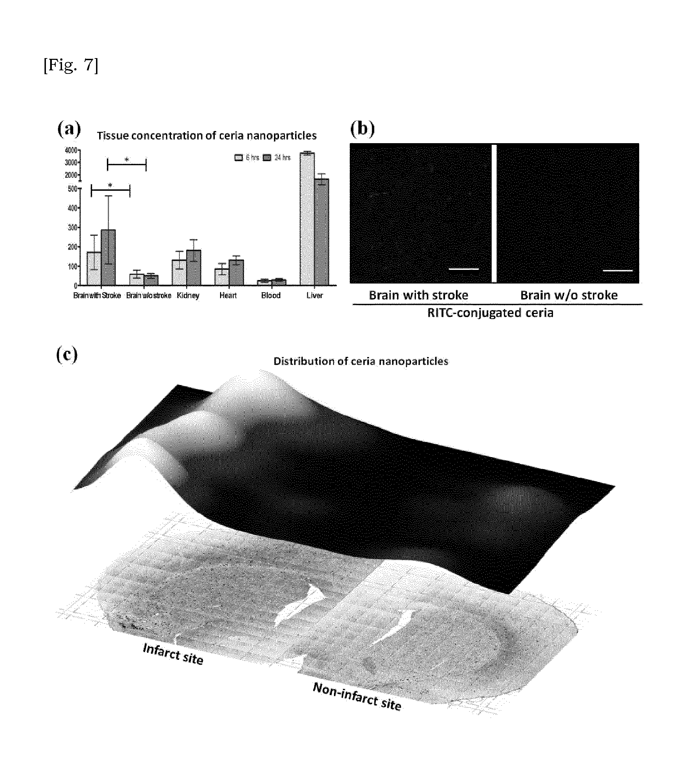

The concentration of the ceria nanoparticles in liver was much higher than those in blood and other internal organs (Fig. 7a). Meanwhile, the concentration in non-ischemic brain was very low, which was strikingly increased 6 or 24 hours after ischemia (p<0.05), and even higher than those in kidney and heart. Then, it was sought to track the ceria nanoparticles microscopically using Rhodamine B Isothiocyanate (RITC)-conjugated ceria nanoparticles and fluorescence microscopic analysis. As shown in Fig. 7b, positively-stained cells were identified to a great extent in the ischemic hemisphere, but scarce in the non-ischemic hemisphere. When a computerized visual augmentation with 3-dimensional reconstruction on the fluorescence signals was conducted, it was found that the signals of ceria nanoparticles were greatly increased in the peri-infarct area in the ischemic hemisphere (Fig. 7c). It was, therefore, demonstrated that intravenously-injected ceria nanoparticles do not sufficiently permeate to the normal brain tissue, but do to the ischemic brain tissue. In fact, it was shown that ceria nanoparticle do not permeate to the normal brain since the diameter of the nanoparticles is larger than the pore size of blood-brain barrier. However, brain ischemia leads to extensive breakage of blood-brain barrier, which may facilitate the pass of ceria nanoparticles to the brain.

### Example 7. Analysis of ROS-scavenging and apoptosis-decreasing effects.

The production of ROS in cerebral ischemia was investigated using hydroethidine (HEt). HEt solution (100 µl, 20 mg/ml in dimethylsulfoxide; Sigma, St. Louis, MO, USA) was administered to male Sprague-Dawley rats (Koatech, Seoul, Republic of Korea) 15 min before ischemia induction. The rats were killed at 4 hours after the ischemia induction by transcardial perfusion. Fluorescence was assessed microscopically at excitation = 355 nm and emission >415 nm for HEt detection or at excitation = 510-550 nm and emission >580 nm for oxidized HEt detection. To evaluate pro-apoptotic proteins, the rats were sacrificed via decaptitation, and the brains were extracted at 24 hours after the induction of focal ischemia-reperfusion. After the centrifugation of hemisphere homogenates, 50 µg of the protein was separated on a 10% sodium dodecyl sulfate-polyacrylamide gel and transferred to nitrocellulose membranes. Theses membranes were incubated in blocking buffer (5% skim milk in 50 mM Tris pH 7.5, 0.15 mM NaCl, 0.05% Tween-20) and the blots were probed with antibodies recognizing phospho-P53, cleaved caspase-3, and gelsolin (Cell Signaling Tech., Danvers, MA). Immunoreactivity was visualized by enhanced chemiluminescence, and the relative densities were determined by comparison of the measured values with the mean values of the control group.

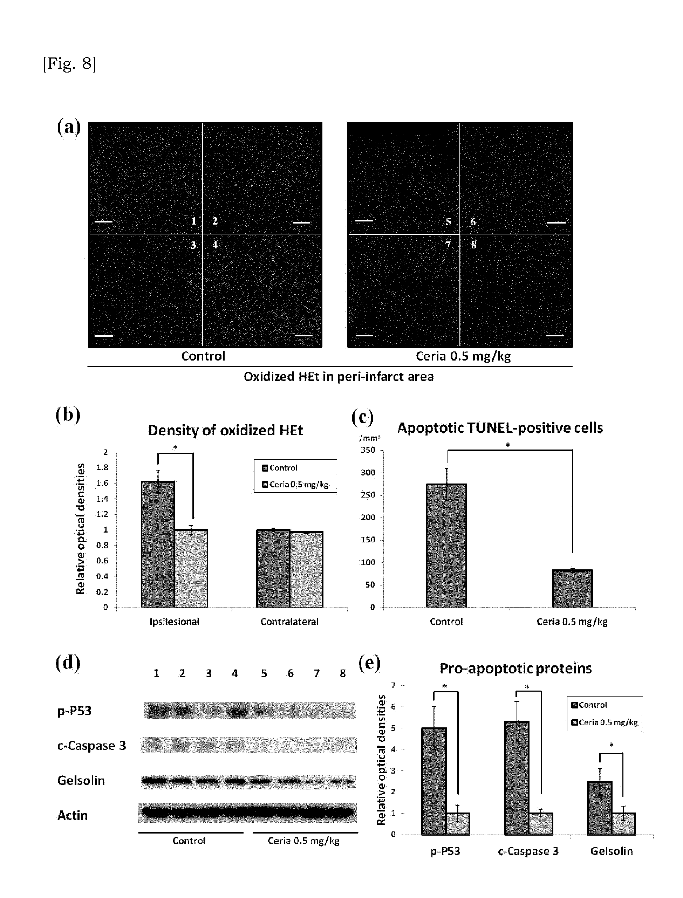

After stroke, the signals of oxidized hydroethidine (detector of ROS) were decreased in ceria-injected group compared with the control (p<0.05; Fig. 8a and Fig. 8b). This result indicates that the ceria nanoparticles properly performed its role as an antioxidant after ischemia. To investigate the effects of ceria nanoparticles for apoptosis after stroke, apoptotic TUNEL-positive cells (subgroup of TUNEL-positive cells according to the morphologic criteria) were analyzed. The number of apoptotic cells in ceria-nanoparticle-injected group was lower than in the control group (*p*<0.05; Fig. 8c), and pro-apoptotic proteins such as phospho-P53, cleaved caspase-3 and gelsolin decreased in the ceria-nanoparticle-injected group (*p*<0.05; Fig. 8d and Fig. 8e). The ROS production and apoptosis are closely related since they are occurred in the same place, mitochondria.

## Claims

1. A ceria nanocomposite comprising a ceria nanoparticle, wherein said ceria nanoparticle is encapsulated with a surfactant and said surfactant is encapsulated with polyethylene glycol-phospholipid.

2. The ceria nanocomposite of Claim 1, wherein a size of said ceria nanoparticle is 3 nm to 20 nm.

3. The ceria nanocomposite of Claim 1, wherein a size of said ceria nanocomposite is 15 nm to 100 nm.

4. The ceria nanocomposite of Claim 1, wherein said surfactant is selected from the group consisted of alkyltrimethylammonium ((CH₃)₃RNX, where R is C₈-C₂₅ and X is Br, Cl or I), C₆-C₂₀ alkylamine, poly(alkylene oxide) triblock copolymer and C₁₂-C₁₈ fatty acid.

5. The ceria nanocomposite of Claim 4, wherein said alkyltrimethylammonium is selected from the group consisting of cetyltrimethylammonium bromide, octyltrimethylammonium bromide and dodecyltrimethylammonium bromide.

6. The ceria nanocomposite of Claim 4, wherein said C₆-C₂₀ alkyl amine is selected from the group consisting of oleylamine, octylamine, hexadecylamine and octadecylamine.

7. The ceria nanocomposite of Claim 4, wherein said poly(alkylene oxide) triblock copolymer is (ethylene oxide)₂₀(propylene oxide)₇₀(ethylene oxide)₂₀.

8. The ceria nanocomposite of Claim 4, wherein said C₁₂-C₁₈ fatty acid is selected from the group consisting of oleic acid, linoleic acid, lauric acid, myristic acid, palmitic acid and stearic acid.

9. A pharmaceutical composition for treating or preventing ischemic stroke, comprising a ceria nanocomposite which comprises a ceria nanoparticle, wherein said ceria nanoparticle is enclosed with a surfactant and said surfactant is capped with polyethylene glycol-phospholipid.

10. The pharmaceutical composition of Claim 9, wherein a size of said ceria nanoparticle is 3 nm to 20 nm.

11. The pharmaceutical composition of Claim 9, wherein a size of said ceria nanocomposite is 15 nm to 100 nm.

12. The pharmaceutical composition of Claim 9, wherein said surfactant is selected from the group consisted of alkyltrimethylammonium ((CH₃)₃RNX, where R is C₈-C₂₅ and X is Br, Cl or I), C₆-C₂₀ alkylamine, poly(alkylene oxide) triblock copolymer and C₁₂-C₁₈ fatty acid.

13. The pharmaceutical composition of Claim 12, wherein said alkyltrimethylammonium is selected from the group consisting of cetyltrimethylammonium bromide, octyltrimethylammonium bromide and dodecyltrimethylammonium bromide.

14. The pharmaceutical composition of Claim 12, wherein said C₆-C₂₀ alkyl amine is selected from the group consisting of oleylamine, octylamine, hexadecylamine and octadecylamine.

15. The pharmaceutical composition of Claim 12, wherein said poly(alkylene oxide) triblock copolymer is (ethylene oxide)₂₀(propylene oxide)₇₀(ethylene oxide)₂₀.

16. The pharmaceutical composition of Claim 12, wherein said C₁₂-C₁₈ fatty acid is selected from the group consisting of oleic acid, linoleic acid, lauric acid, myristic acid, palmitic acid and stearic acid.

17. The pharmaceutical composition of Claim 9, wherein the contents of said ceria nanocomposite is 0.5 mg/kg - 0.7 mg/kg.

18. The pharmaceutical composition of Claim 9, wherein an administration route of said pharmaceutical composition is selected from the group consisting of oral, nasal, intravenous, rectal, intraperitoneal, subcutaneous and intracutaneous administrations.

19. A method for preparing a ceria nanocomposite, comprising:
(i) reacting a cerium precursor with a surfactant in a first organic solvent to form a cerium-surfactant complex;
(ii) adding water to said cerium-surfactant complex solution of the step (i), while heating said solution, to form a cerium nanoparticle enclosed with said surfactant; and
(iii) encapsulating said surfactant-capped ceria nanoparticle with polyethylene glycol-lipid in a second organic solvent.

20. The method of Claim 19, wherein said first solvent is selected from the group consisting of xylene, toluene, mesitylene, octyl ether, butyl ether, hexyl ether, decyl ether, pyridine, dimethyl sulfoxide, dimethylformamide, octanol, decanol, octane, decane, dodecane, tetradecane and hexadecane.

21. The method of Claim 19, wherein said cerium precursor is selected from the group consisting of cerium (III) acetate hydrate, cerium (III) acetylacetonate hydrate, cerium (III) carbonate hydrate, cerium (IV) hydroxide, cerium (III) fluoride, cerium (IV) fluoride, cerium (III) chloride, cerium (III) chloride heptahydrate, cerium (III) bromide, cerium (III) iodide, cerium (III) nitrate hydrate, cerium (III) oxalate hydrate, cerium (III) sulfate, cerium (III) sulfate hydrate and cerium (IV) sulfate.

22. The method of Claim 19, wherein said said surfactant is selected from the group consisted of alkyltrimethylammonium ((CH₃)₃RNX, where R is C₈-C₂₅ and X is Br, Cl or I), C₆-C₂₀ alkylamine, poly(alkylene oxide) triblock copolymer and C₁₂-C₁₈ fatty acid.

23. The method of Claim 22, wherein said alkyltrimethylammonium is selected from the group consisting of cetyltrimethylammonium bromide, octyltrimethylammonium bromide and dodecyltrimethylammonium bromide.

24. The method of Claim 22, wherein said C₆-C₂₀ alkyl amine is selected from the group consisting of oleylamine, octylamine, hexadecylamine and octadecylamine.

25. The method of Claim 22, wherein said poly(alkylene oxide) triblock copolymer is (ethylene oxide)₂₀(propylene oxide)₇₀(ethylene oxide)₂₀.

26. The method of Claim 22, wherein said C₁₂-C₁₈ fatty acid is selected from the group consisting of oleic acid, linoleic acid, lauric acid, myristic acid, palmitic acid and stearic acid.

27. The method of Claim 19, wherein a heating temperature of the step (ii) is 80°C - 90°C.

28. The method of Claim 19, wherein a heating time of the step (ii) is 30 min - 3 hr.

29. The method of Claim 19, wherein said second organic solvent is selected from the group consisting of chloroform, dichloromethane, pentane, hexane, cyclohexane, ethyl acetate, tetrahydrofuran, diethyl ether and trichloroethylene.

30. The method of Claim 19, wherein a size of said polyethylene glycol-capped ceria nanoparticle is 15 nm - 100 nm.
